# EUROPÄISCHE PATENTSCHRIFT

(11) **EP 0 929 629 B1**
(45) Veröffentlichungstag und Bekanntmachung des Hinweises auf die Patenterteilung: **16.01.2002**
(21) Anmeldenummer: 97910331.4
(22) Anmeldetag: 23.09.1997
(51) Int. Cl.: C11D 1/65, C11D 1/52, A61K 7/50, B01F 17/00

(54) **VERWENDUNG VON N-(3-DIALKYLAMINO)-PROPYL-N-POLYHYDROXYALKYL-CARBONSÄUREAMIDEN ALS VERDICKUNGSMITTEL FÜR FLÜSSIGE WÄSSRIGE TENSIDSYSTEME**
USE OF N-(3-DIALKYLAMINO)-PROPYL-N-POLYHYDROXYALKYL CARBOXYLIC ACID AMIDES AS THICKENERS FOR LIQUID AQUEOUS SURFACTANT SYSTEMS
UTILISATION D'AMIDES DE L'ACIDE N-(3-DIALKYLAMINO)-PROPYL-N-POLYHYDROXYALKYL-CARBOXYLIQUE COMME EPAISSISSANTS POUR DES SYSTEMES TENSIO-ACTIFS LIQUIDES AQUEUX

(30) Priorität: 30.09.1996 DE 19640185
(43) Veröffentlichungstag der Anmeldung: 21.07.1999
(73) Patentinhaber: Clariant GmbH, 65929 Frankfurt am Main (DE)
(72) Erfinder: TUROWSKI, Angelika, D-65777 Kelkheim (DE); LÖFFLER, Matthias, D-65527 Niedernhausen (DE); SCHOLZ, Hans, Jürgen, D-63755 Frankfurt (DE); SKRYPZAK, Werner, D-65719 Hofheim (DE); PAPENFUHS, Bernd, D-84524 Neuöttingen (DE)
(86) Internationale Anmeldenummer: EP9705200
(87) Internationale Veröffentlichungsnummer: WO9814543

(56) Entgegenhaltungen:
- EP-A- 0 176 151
- EP-A- 0 285 768
- EP-A- 0 729 747
- DE-A- 19 512 299

## Beschreibung

Die Produktion flüssiger Produkte auf dem Kosmetik- und Waschmittelsektor nimmt ständig zu. Speziell im Bereich der Körperreinigungsmittel sind es die flüssigen Haarshampoos, Schaumbäder und Duschbäder, die in den letzten Jahren zunehmend an Bedeutung gewonnen haben. Flüssige Geschirrspülmittel und flüssige Feinwaschmittel haben sich ebenfalls einen festen Platz auf dem Markt erobert.

Voraussetzung für eine brauchbare flüssige Tensidrezeptur ist eine gute Lagerbeständigkeit. Die Flüssigkeit darf bei Temperaturschwankungen nicht eintrüben bzw. Niederschläge bilden. Außerdem wird eine gute Hautverträglichkeit verlangt. Das Produkt sollte die Haut möglichst nicht entfetten bzw. sollte nicht zu Hautreizungen führen.

Das flüssige Tensidsystem muß jedoch vor allem auch eine Viskosität haben, die dem jeweiligen Verwendungszweck angepaßt ist und sich möglichst variabel einstellen läßt.
So ist die Viskosität ein entscheidendes Kriterium für die Qualität eines flüssigen Tensidpräparates. Es werden z.B. von einem Duschgel sehr hohe Viskositäten gefordert, während ein Haarshampoo gewöhnlich eine fließfähige Flüssigkeit mit einer relativ niedrigen Viskosität von 1000 bis 4000 mPas darstellt.

Bekannte Verdickungsmittel für flüssige Tensidformulierungen sind unter anderem nichtionogene Fettsäurepolyalkylenglykolester, wie ANTIL (Molgewicht ca. 3000, Goldschmidt AG), sowie seit vielen Jahren nichtionische Fettsäurealkanolamide (vergl. J. Amer. Oil Chem. Soc. 35, 548 (1958)). Als Fettsäurealkanolamid wird in der Praxis bevorzugt das Kokosfettsäurediethanolamid (SUPERAMID) eingesetzt. Es zeigt gegenüber anderen Fettsäurediethanolamiden die besten Verdickungseigenschaften.

Der Grad der Verdickung hängt stark vom Tensidsystem und vom Elektrolytzusatz ab. So ist z.B. bekannt, daß sek.-Paraffinsulfonate als Tenside in flüssigen Einstellungen Schwierigkeiten bei der Viskositätseinstellung bereiten. Die bekannten, marktgängigen o.a. Verdickungsmittel zeigen bei sek.- Paraffinsulfonaten auch in Gegenwart von Elektrolyten keine ausreichenden Verdickungseffekte. EP-A-0 285 768 beschreibt die Verwendung von N-Polyhydroxyalkylfettsäureamiden als Verdickungsmittel für flüssige wässrige Tensidsysteme, die insbesondere bei Anwesenheit von sek.-Paraffinsulfonaten eine gute Wirkung erbringen.

Ferner ist bekannt, daß sich wässrige, Fettalkoholethersulfate enthaltende Tensidformulierungen, wie sie in kosmetischen Formulierungen oft Anwendung finden, durch Zusatz von Alkylamidobetainen hinsichtlich einer gewünschten Viskosität eingestellt werden können. Alkylamidobetaine weisen als Nebenprodukte jedoch einen hohen Natriumchloridgehalt auf, der häufig zu Korrosionen bei der Lagerung bzw. Verarbeitung führen kann.

Fettsäure-N-Alkyl-polyhydroxyalkylamide und insbesondere Fettsäure-N-methylglucamide stellen nichtionische Tenside dar, die wegen ihres guten anwendungstechnischen Profils beispielsweise zur Herstellung von Wasch- und Reinigungsmitteln eingesetzt werden. Die Verwendung dieser Stoffe ist Gegenstand einer Vielzahl von Veröffentlichungen. Aus EP-A-0 285 768 ist beispielsweise ihr Einsatz als Verdickungsmittel in wäßrigen Reinigungsmittelsystemen beschrieben. Nachteilig für die Anwendung und Formulierbarkeit ist eine begrenzte Löslichkeit, insbesondere ab einer Kettenlänge größer als C₁₆. Bei höheren Konzentrationen in Wasser sind sie aufgrund der hohen Viskosität nur schwer zu handhaben. Höhere Temperaturen zur Absenkung der Viskosität führen zu verstärkter Hydrolyse. WO-A-974751, die unter Artikel 54(3) EP Ü fällt, bercheibt Säure-Addukle von Fettsäurepolyhydrokyalkylamiden, Kompositionen, die diese Säure-Addukle enthalten und die Verwendung dieser Saure-Addukle und Kompositionen als Schaum - und Verdickungsmittel.

Es bestand somit die Aufgabenstellung, Verdicker für flüssige wäßrige Tensidsysteme zu finden, welche unter den unterschiedlichen Anforderungen der Praxis in Hinblick auf Tensid, Elektrolyt und Einsatzzweck genügen.

Überraschenderweise wurde gefunden, daß Verbindungen der Formel 1, wie sie in DE-A-195 12 299.2 beschrieben werden, sich als Verdicker eignen.

Gegenstand der Erfindung ist die Verwendung von Verbindungen der Formel 1 als Verdickungsmittel für flüssige, wäßrige Tensidsysteme 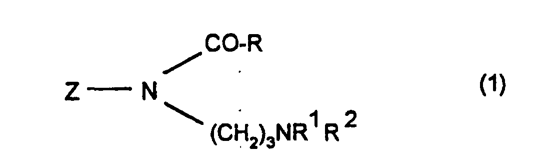 worin R ein aliphatischer Rest mit 8 bis 24 C-Atomen ist, R¹ und R², die gleich oder verschieden sind, Alkyl mit 1 bis 4 C-Atomen oder Hydroxyalkyl mit 2 bis 4 C-Atomen und Z einen linearen Polyhydroxykohlenwasserstoffrest mit mindestens 3 OH-Gruppen, die auch alkoxyliert sein können, bedeuten.

Ein wesentlicher Vorteil der erfindungsgemäß einzusetzenden Verbindungsklasse ist ihre hohe Anpassungsfähigkeit an die jeweiligen Bedürfnisse des Tensidsystems durch die Variationsmöglichkeiten der Substituenten Z, R, R¹ und R². So lassen sich in der erfindungsgemäßen Stoffklasse neben neben der Fettsäurealkylkette R die hydrophobierenden Substituenten am Stickstoff, R¹ und R², sowie der hydrophilierende Kohlenhydratrest Z variieren. Zudem ist durch den basischen Dialkylaminoalkylen-Rest eine gezielte Einflußnahme auf die Eigenschaften durch Einstellung eines bestimmten pH-Wertes möglich.

Bevorzugt sind Verbindungen der Formel 1, in denen R Fettalkyl, R¹ und R² Methyl und Z der Rest eines Zuckeralkohols ist, der sich von einem reduzierenden Mono- oder Disaccharid, insbesondere von der Glucose, ableitet.

Die Aufgabe der vorliegenden Erfindung bestand in der Entwicklung von Verdickungsmitteln für flüssige wässrige Tensidmischungen durch Verwendung von N-(3-Dialkylamino)-propyl-N-polyhydroxyalkylcarbonsäureamiden die neben verbesserten anwendungstechnischen Eigenschaften, wie gute Wasserlöslichkeit, hohe Tensidkonzentration ohne organische Lösungsmittel, geringere Schmelzpunkte, günstige Viskositätseinstellungen unter den unterschiedlichen Anforderungen der Praxis in Hinblick auf Tensid, Elektrolyt und Einsatzzweck ermöglichen, ohne Leistungseinbußen in der Reinigungswirkung der Detergensgemische zu bewirken.

Die erfindungsgemäßen Formulierungen können eine Verbindung der Formel 1 als alleiniges Tensid enthalten, vorzugsweise werden diese Tenside kombiniert mit weiteren üblichen anionischen, nicht ionischen, kationischen und/oder amphoteren Tensiden. Das Mischungsverhältnis zwischen den Tensiden der Formel 1 und den übrigen Tensiden kann in breiten Grenzen schwanken, etwa im Gewichtsverhältnis von 1 zu 99 bis 99 zu 1, vorzugsweise von 80 zu 20 bis 20 zu 80. Die Gesamtkonzentration an Tensiden in den erfindungsgemäßen Formulierungen kann 1 bis 99, vorzugsweise 5 bis 50 Gew.-% betragen. Vorzugsweise sind die Verbindungen der Formel (1) in einer Menge von 0.5 bis 30 Gewichtsprozent, bezogen auf das gesamte flüssige Tensidsystem, in dem Tensidsystem enthalten.

Als anionische Tenside kommen in Betracht Sulfonate, Sulfate, Carboxylate, Phosphate und Mischungen aus den genannten Verbindungen. Geeignete Kationen sind hierbei Alkalimetalle, wie z.B. Natrium oder Kalium oder Erdalkalimetalle, wie z.B. Calcium oder Magnesium sowie Ammonium, substituierte Ammoniumverbindungen, einschließlich Mono-, Di- oder Triethanolammoniumkationen und Mischungen der Kationen. Folgende Typen von anionischen Tensiden sind von besonderem Interesse:
Alkylestersulfonate, Alkylsulfate, Alkylethersulfate, Alkylbenzolsulfonate, sekundäre Alkansulfonate, Seifen wie im folgenden beschrieben.

Alkylestersulfonate sind unter anderem lineare Ester von C₈-C₂₀-Carboxylsäuren (d.h. Fettsäuren), welche mittels gasförmigem SO₃ sulfoniert werden, wie in "The Journal of the American Oil Chemists Society" 52 (1975), pp. 323-329 beschrieben wird. Geeignete Ausgangsmaterialien sind natürliche Fette wie z.B. Talg, Palmöl oder Cocosöl, können aber auch synthetischer Natur sein. Bevorzugte Alkylestersulfonate speziell für Waschmittelanwendungen, sind Verbindungen der Formel 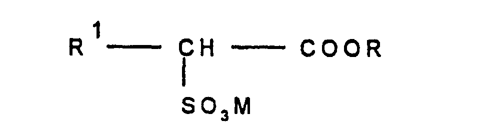 worin R¹ einen C₈-C₂₀-Kohlenwasserstoffrest, bevorzugt Alkyl und R einen C₁-C₆ Kohlenwasserstoffrest, bevorzugt Alkyl, darstellt. M steht für ein Kation, das ein wasserlösliches Salz mit dem Alkylestersulfonat bildet. Geeignete Kationen sind Natrium, Kalium, Lithium oder Ammoniumkationen, wie Monoethanolamin, Diethanolamin und Triethanolamin. Bevorzugt bedeuten R¹ C₁₀-C₁₆-Alkyl und R Methyl, Ethyl oder Isopropyl. Besonders bevorzugt sind Methylestersulfonate, in denen R¹ C₁₀-C₁₆-Alkyl bedeutet.

Alkylsulfate sind hier wasserlösliche Salze oder Säuren der Formel ROSO₃M, worin R bevorzugt ein C₁₀-C₂₄-Kohlenwasserstoffrest, bevorzugt C₁₀-C₂₀-Alkyl oder Hydroxyalkyl, besonders bevorzugt C₁₂-C₁₈ Alkyl- oder Hydroxyalkyl darstellt. M ist Wasserstoff oder ein Kation, z.B. ein Alkalimetallkation (z.B. Natrium, Kalium, Lithium), Ammonium oder substituiertes Ammonium z.B. Methyl-, Dimethyl- und Trimethylammoniumkationen und quaternäre Ammoniumkationen, wie Tetramethylammonium- und Dimethylpiperidiniumkationen und quaternäre Ammoniumkationen, abgeleitet von Alkylaminen wie Ethylamin, Diethylamin, Triethylamin und Mischungen davon. Alkylketten mit C₁₂-C₁₆ sind für niedrige Waschtemperaturen (z.B. unter ca. 50°C) und Alkylketten mit C₁₆-C₁₈ für höhere Waschtemperaturen (z.B. oberhalb ca. 50°C) bevorzugt.

Alkylethersulfate sind wasserlösliche Salze oder Säuren der Formel RO(A)ₘ SO₃M, worin R einen unsubstituierten C₁₀-C₂₄-Alkyl- oder Hydroxyalkylrest, bevorzugt einen C₁₂-C₂₀ Alkyl- oder Hydroxyalkylrest, besonders bevorzugt C₁₂-C₁₈-Alkyl- oder Hydroxyalkylrest darstellt. A ist eine Ethoxy- oder Propoxyeinheit, m ist eine Zahl größer als 0, vorzugsweise zwischen 0,5 und 6, besonders bevorzugt zwischen 0,5 und 3 und M ist ein Wasserstoffatom oder ein Kation wie z.B. Natrium, Kalium, Lithium, Calcium, Magnesium, Ammonium oder ein substituiertes Ammoniumkation. Spezifische Beispiele von substituierten Ammoniumkationen sind Methyl-, Dimethyl-, Trimethylammonium- und quatemäre Ammoniumkationen wie Tetramethylammonium und Dimethylpiperidiniumkationen, sowie solche, die von Alkylaminen, wie Ethylamin, Diethylamin, Triethylamin, Mischungen davon abgeleitet sind. Als Beispiele seien C₁₂-C₁₈-Fettalkoholethersulfate genannt, wobei der Gehalt an Ethylenoxid 1, 2, 2.5, 3 oder 4 mol pro mol Fettalkoholethersulfat beträgt, und in denen M Natrium oder Kalium ist. Ein bevorzugtes Ethersulfat ist Lauroylethersulfat.

In sekundären Alkansulfonaten kann die Alkylgruppe entweder gesättigt oder ungesättigt, verzweigt oder linear und gegebenenfalls mit einer Hydroxylgruppe substituiert sein. Die Sulfogruppe kann eine beliebige Position an der gesamten C-Kette einnehmen, wobei die primären Methylgruppen am Kettenanfang und Kettenende keine Sulfogruppen besitzen. Die bevorzugten sekundären Alkansulfonate enthalten lineare Alkylketten mit ca. 9 bis 25 Kohlenstoffatomen, bevorzugt von ca. 10 bis ca. 20 Kohlenstoffatome und besonders bevorzugt ca. 13 bis 17 Kohlenstoffatome. Als Kation ist beispielsweise Natrium, Kalium, Ammonium, Mono-, Di- oder Triethanolammonium, Calcium oder Magnesium bevorzugt.

Weitere geeignete anionische Tenside sind Alkenyl- oder Alkylbenzolsulfonate. Die Alkenyl- oder Alkylgruppe kann verzweigt oder linear und gegebenenfalls mit einer Hydroxylgruppe substituiert sein. Die bevorzugten Alkylbenzolsulfonate enthalten lineare Alkylketten mit 9 bis 25 Kohlenstoffatomen, bevorzugt von 10 bis 13 Kohlenstoffatome, das Kation ist Natrium, Kalium, Ammonium, Mono-, Di- oder Triethanolammonium, Calcium oder Magnesium und Mischungen davon. Für milde Tensidsysteme ist Magnesium als Kation bevorzugt, für Standardwaschanwendungen dagegen Natrium. Gleiches gilt für Alkenylbenzolsulfonate

Der Begriff anionische Tenside schließt auch Olefinsulfonate mit ein, die durch Sulfonierung von C₁₂-C₂₄-, vorzugsweise C₁₄-C₁₆-α-Olefinen mit Schwefeltrioxid und anschließende Neutralisation erhalten werden. Bedingt durch das Herstellverfahren, können diese Olefinsulfonate kleinere Mengen an Hydroxyalkansulfonaten und Alkandisulfonaten enthalten. Spezielle Mischungen von α-Olefinsulfonaten sind in US-3,332,880 beschrieben.

Weitere bevorzugte anionische Tenside sind Carboxylate, z.B. Fettsäureseifen und vergleichbare Tenside. Die Seifen können gesättigt oder ungesättigt sein und können verschiedene Substituenten, wie Hydroxylgruppen oder α-Sulfonatgruppen enthalten. Bevorzugt sind lineare gesättigte oder ungesättigte Kohlenwasserstoffreste als Anteil mit 6 bis 30, bevorzugt 10 bis 18 Kohlenstoffatomen.

Als anionische Tenside kommen weiterhin Salze von Acylaminocarbonsäuren in Frage, die durch Umsetzung von Fettsäurechloriden mit Natriumsarkosinat im alkalischen Medium entstehenden Acylsarcosinate; Fettsäure-Eiweiß-Kondensationsprodukte, die durch Umsetzung von Fettsäurechloriden mit Oligopeptiden erhalten werden; Salze von Alkylsulfamidocarbonsäuren, Salze von Alkyl- und Alkylarylethercarbonsäuren; C₈-C₂₄-Olefinsulfonate, sulfonierte Polycarboxylsäuren, hergestellt durch Sulfonierung der Pyrolyseprodukte von Erdalkalimetallcitraten, wie z.B. beschrieben in GB-1,082,179; Alkylglycerinsulfate, Fettacylglycerinsulfate, Alkylphenolethersulfate, primäre Paraffinsulfonate, Alkylphosphate, Alkyletherphosphate, Isethionate, wie Acylisethionate, N-Acyltauride, Alkylsuccinate, Sulfosuccinate, Monoester der Sulfosuccinate (besonders gesättigte und ungesättigte C₁₂-C₁₈-Monoester) und Diester der Sulfosuccinate (besonders gesättigte und ungesättigte C₁₂-C₁₈-Diester), Acylsarcosinate, Sulfate von Alkylpolysacchariden wie Sulfate von Alkylpolyglycosiden, verzweigte primäre Alkylsulfate und Alkylpolyethoxycarboxylate wie die der Formel RO(CH₂CH₂)ₖCH₂COO⁻M⁺, worin R C₈-C₂₂-Alkyl, k eine Zahl von 0 bis 10 und M ein Kation ist, Harzsäuren oder hydrierte Harzsäuren, wie Rosin oder hydriertes Rosin oder Tallölharze und Tallölharzsäuren. Weitere Beispiele sind in "Surface Active Agents and Detergents" (Vol. I und II, Schwanz, Perry und Berch) beschrieben.

Als nicht-ionische Tenside kommen beispielsweise folgende Typen in Frage:

Polyethylen-, Polypropylen- und Polybutylenoxidkondensate von Alkylphenolen.

Diese Verbindungen umfassen die Kondensationsprodukte von Alkylphenolen mit einer C₆-C₂₀-Alkylgruppe, die entweder linear oder verzweigt sein kann, mit Alkenoxiden. Bevorzugt sind Verbindungen mit ca. 5 bis 25 mol Ethylenoxid pro mol Alkylphenol. Kommerziell erhältliche Tenside diesen Typs sind z.B. Igepal® CO-630, Triton® X-45, X-114, X-100 und X102, und die ® Arkopal-N-Marken der Hoechst AG.

Kondensationsprodukte von aliphatischen Alkoholen mit ca. 1 bis ca. 25 mol Ethylenoxid.

Die Alkylkette der aliphatischen Alkohole kann linear oder verzweigt, primär oder sekundär sein, und enthält im allgemeinen 8 bis 22 Kohlenstoffatome. Besonders bevorzugt sind die Kondensationsprodukte von C₁₀-C₂₀-Alkoholen, mit 2 bis 18 mol Ethylenoxid pro mol Alkohol. Die Alkylkette kann gesättigt oder auch ungesättigt sein. Die Alkoholethoxilate können eine enge ("Narrow Range Ethoxylates") oder eine breite Homologenverteilung des Ethylenoxides ("Broad Range Ethoxylates") aufweisen. Beispiele von kommerziell erhältlichen nichtionischen Tensiden dieses Types sind Teritol® 15-S-9 (Kondensationsprodukt eines C₁₁-C₁₅ linearen sekundären Alkohols mit 9 mol Ethylenoxid), Tergitol® 24-L-NMW (Kondensationsprodukt eines C₁₂-C₁₄-linearen primären Alkohols mit 6 mol Ethylenoxid mit enger Molgewichtsverteilung). Ebenfalls unter diese Produktklasse fallen die Genapol® -Marken der Hoechst AG.

Kondensationsprodukte von Ethylenoxid mit einer hydrophoben Basis, gebildet durch Kondensation von Propylenoxid mit Propylenglykol.

Der hydrophobe Teil dieser Verbindungen weist bevorzugt ein Molekulargewicht zwischen ca. 1500 und ca. 1800 auf. Die Anlagerung von Ethylenoxid an diesen hydrophoben Teil führt zu einer Verbesserung der Wasserlöslichkeit. Das Produkt ist flüssig bis zu einem Polyoxyethylengehalt von 50 % des Gesamtgewichtes des Kondensationsproduktes, was einer Kondensation mit bis zu 40 mol Ethylenoxid entspricht. Kommerziell erhätliche Beispiele dieser Produktklasse sind die Pluronic® -Marken der BASF und die ® Genapol PF-Marken der Hoechst AG.

Kondensationsprodukt von Ethylenoxid mit einem Reaktionsprodukt von Propylenoxid und Ethylendiamin.

Die hydrophobe Einheit diese Verbindungen besteht aus dem Reaktionsprodukt von Ethylendiamin mit überschüssigem Propylenoxid und weist im allgemeinen ein Molekulargewicht von 2500 bis 3000 auf. An diese hydrophobe Einheit wird Ethylenoxid addiert, bis das Produkt einen Gehalt von 40 bis 80 Gew.-% Polyoxyethylen und ein Molekulargewicht von 5000 bis 11000 aufweist. Kommerziell erhältliche Beispiele dieser Verbindungsklase sind die ® Tetronic-Marken der BASF und die ® Genapol PN-Marken der Hoechst AG.

### Semipolare nichtionische Tenside

Diese spezielle Kategorie von nichtionischen Verbindungen umfaßt wasserlösliche Aminoxide, wasserlösliche Phosphinoxide und wasserlösliche Sulfoxide jeweils mit einem Alkylrest von ca. 10 bis ca. 18 Kohlenstoffatomen. Semipolare nichtionische Tenside sind auch Aminoxide der Formel 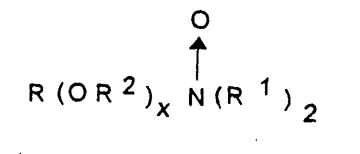 R ist hierbei eine Alkyl-Hydroxyalkyl- oder Alkylphenolgruppe mit jeweils 8 bis 22 Kohlenstoffatomen, R² ist eine Alkylen- oder Hydroxyalkylengruppe mit 2 bis 3 Kohlenstoffatomen oder Mischungen hiervon, jeder Rest R¹ ist eine Alkyl- oder Hydroxyalkylgruppe mit 1 bis 3 Kohlenstoffatomen oder eine Polyethylenoxidgruppe mit 1 bis 3 Ethylenoxideinheiten. Die R¹-Gruppen können miteinander über ein Sauerstoff- oder Stickstoffatom verbunden sein und somit einen Ring bilden. Aminoxide dieser Art sind besonders C₁₀-C₁₈-Alkyldimethylaminoxide und C₈-C₁₂-Alkoxiethyl-Dihydroxyethylaminoxide.

### Fettsäureamide

Fettsäureamide besitzen die Formel 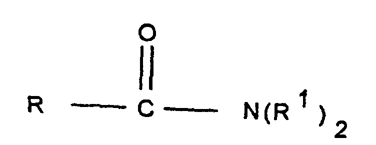 worin R eine Alkylgruppe mit 7 bis 21, bevorzugt 9 bis 17 Kohlenstoffatomen ist und jeder Rest R¹ Wasserstoff, C₁-C₄-Alkyl, C₁-C₄-Hydroxyalkyl oder (C₂H₄O)ₓH bedeutet, wobei x von 1 bis 3 variiert. Bevorzugt sind C₈-C₂₀-Amide, -monoethanolamide, -diethanolamide und -isopropanolamide.

Weitere geeignete nicht-ionische Tenside sind insbesondere Alkyl- und Alkenyloligoglycoside sowie Fettsäurepolyglykolester oder Fettaminpolyglykolester mit jeweils 8 bis 20, vorzugsweise 12 bis 18 C-Atomen im Fettalkylrest, alkoxylierte Triglycamide, Mischether oder Mischformyle, Fettsäure-N-alkylglucamide, Proteinhydrolysate, Phosphinoxide oder Dialkylsulfoxide.

Typische Beispiele für amphotere bzw. zwitterionische Tenside sind Alkylbetaine, Alkylamidbetaine, Aminopropionate, Aminoglycinate, oder amphotere Imdiazolinium-Verbindungen der Formel 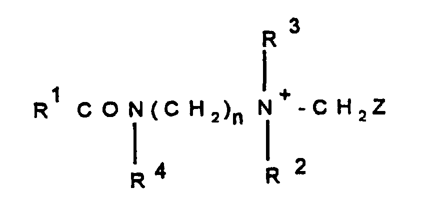 worin R¹ C₈-C₂₂-Alkyl- oder -Alkenyl, R² Wasserstoff oder CH₂CO₂M, R³ CH₂CH₂OH oder CH₂CH₂OCH₂CH₂COOM, R⁴ Wasserstoff, CH₂CH₂OH oder CH₂CH₂COOM, Z CO₂M oder CH₂CO₂M, n 2 oder 3, bevorzugt 2, M Wasserstoff oder ein Kation wie Alkalimetall, Erdalkalimetall, Ammoniak oder Alkanolammonium bedeutet.

Bevorzugte amphotere Tenside dieser Formel sind Monocarboxylate und Dicarboxylate. Beispiele hierfür sind Cocoamphocarboxypropionat, Cocoamidocarboxypropionsäure, Cocoamphocarboxyglycinat (auch als Cocoamphodiacetat bezeichnet) und Cocoamphoacetat.

Weitere bevorzugte amphotere Tenside sind Alkyldimethylbetaine und Alkyldipolyethoxybetaine mit einem Alkylrest, der linear oder verzweigt sein kann, mit 8 bis 22 Kohlenstoffatomen, bevorzugt mit 8 bis 18 Kohlenstoffatomen und besonders bevorzugt mit 12 bis 18 Kohlenstoffatomen. Diese Verbindungen werden z.B. von der Hoechst AG unter dem Handelnamen ® Genagen LAB vermarktet.

Geeignete kationische Tenside sind substituierte oder unsubstituierte geradkettige oder verzweigte quartäre Ammoniumsalze vom Typ R¹N(CH₃)₃^{⊕}X^{⊖}, R¹R²N(CH₃)₂^{⊕}X^{⊖}, R¹R²R³N(CH₃)^{⊕}X^{⊖} oder R¹R²R³R⁴N^{⊕}X^{⊖}. Die Reste R^{1,} R^{2,} R³ und R⁴ können vorzugsweise unabhängig voneinander unsubstituiertes Alkyl mit einer Kettenlänge zwischen 8 und 24 C-Atomen, insbesondere zwischen 10 und 18 C-Atomen, Hydroxyalkyl mit 1 bis 4 C-Atomen, Phenyl, C₂- bis C₁₈-Alkenyl, C₇- bis C₂₄-Aralkyl, (C₂H₄O)ₓH, wobei x von 1 bis 3 bedeutet, ein oder mehrere Estergruppen enthaltende Alkylreste oder cyclische quartäre Ammoniumsalze sein. X ist ein geeignetes Anion.

Die erfindungsgemäßen Formulierungen enthalten, je nach Anwendungszweck, neben den genannten Tensiden noch die jeweils spezifischen Hilfs- und Zusatzstoffe. So enthalten Wasch- und Reinigungsformulierungen beispielsweise Builder, Salze, Bleichmittel, Bleichaktivatoren, optische Aufheller, Vergrauungsinhibitoren, Lösungsvermittler und Enzyme.

Übliche Builder sind Natriumaluminiumsilikate (Zeolithe), Schichtsilikate, Phosphate, Phosphonate, Ethylendiamintetraessigsäure, Nitrilotriacetat, Citronensäure und/oder Polycarboxylate.

Als Salze bzw. Stellmittel kommen beispielsweise Natriumsulfat, Natriumcarbonat oder Natriumsilikat (Wasserglas) in Betracht. Als typische Einzelbeispiele für weitere Zusatzstoffe sind Natriumborat, Stärke, Saccharose, Polydextrose, TAED, Stilbenverbindungen, Methylcellulose, Toluolsulfonat, Cumolsulfonat, langkettige Seifen, Silicone, Mischether, Lipasen und Proteasen zu nennen.

In kosmetischen oder pharmazeutischen Formulierungen können neben den genannten Tensiden unter anderem Verdicker, Feuchtigkeitsmittel, Konditioniermittel, Perlglanzmittel, Konservierungsmittel, Parfüm oder Farbstoffe enthalten sein.

Haarshampoos, Haarlotionen oder Dusch- und Schaumbäder können als weitere Hilfs-und Zusatzstoffe Emulgatoren wie etwa alkoxylierte Fettalkohole oder Sorbitanester enthalten.

Als Überfettungsmittel können Substanzen wie beispielsweise polyoxethylierte Lanolinderivate, Lecithinderivate und Fettsäurealkanolamide verwendet werden, wobei die letzteren gleichzeitg als Schaumstabilisatoren dienen.

Geeignete Verdickungsmittel sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und dieester von Fettsäuren, Polyacrylate, Polyvinylalkohol und Polyvinylpyrrolidon sowie Elektrolyte wie Kochsalz und Ammoniumchlorid.

Unter biogenen Wirkstoffen sind beispielsweise Pflanzenextrakte und Vitaminkomplexe zu verstehen.

Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen.

Eine weitere Komponente, die in den Formulierungen, enthalten sein kann, sind nicht flüchtige, flüssige Silicone. Dieses kann entweder ein Polyalkylsiloxan, ein Polyarylsiloxan, ein Polyalkylarylsiloxan oder ein Polyethersiloxan-Copolymer sein und wird in einer Menge von ca. 0,1 % bis ca. 10,0 %, bevorzugt in einer Menge von ca. 0,5 % bis ca. 5,0 % eingesetzt. Mischungen dieser Flüssigkeiten können ebenso verwendet werden und sind auch für bestimmte Anwendungen von Vorteil. Die dispergierten Siliconteilchen sollten unlöslich in der Shampoomatrix sein. Die wichtigsten nicht flüchtigen Polyalkylsiloxane, die eingesetzt werden können, sind z.B. Polydimethylsiloxane mit Viskositäten von 5 bis 600.000 Centistokes, vorzugsweise von 350 und 100.000 Centistokes, bei 25°C. Ein geeignetes, im wesentlichen nicht flüchtiges Polyethersiloxan, ist z.B. ein mit Polypropylenoxid modifiziertes Dimethylpolysiloxan. Es können auch Addukte mit Ethylenoxid und/oder Propylenoxid eingesetzt werden.

Geeignete Silicone werden beispielsweise in US-2,826,551, US-3,946,500, US-4,364,837 und in GB-849,433 beschrieben.

Weiterhin kann Silicon-Gum erfindungsgemäß eingesetzt werden. Silicon-Gums sind in US-4,152,416 beschrieben. Geeignete Silicon-Gums werden ebenso beschrieben in den Produktdatenblättem SE 30, SE 33, SE 54 und SE 76 der Firma General Electric. "Silicon Gum" bedeutet hochmolekulare Polydiorganosiloxane mit einer Molmasse von ca. 200.000 bis 1.000.000. Spezielle Beispiele sind Polydimethylsiloxan, (Polydimethylsiloxan)(Methylvinylsiloxan)-Copolymer, Poly(dimethylsiloxan)(Diphenyl)(Methylvinylsiloxan)-Copolymer und ihre Mischungen, Mischungen von Siliconflüssigkeiten und Silicon-Gums sind ebenfalls geeignet.

Als Konservierungsmittel eignen sich beispielsweise Phenoxyethanol, Formaldehydlösung, Parabene, Pentadiol oder Sorbinsäure.

Als Perlglanzmittel kommen beispielsweise Glycoldistearinsäureester wie Ethylenglycoldistearat, aber auch Fettsäuremonoglycolester in Betracht.

Als Farbstoffe können die für kosmetischen Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung eingesetzt.

Der Gesamtanteil der Hilfs- und Zusatzstoffe kann 1 bis 50, vorzugsweise 5 bis 40 Gew.-%, bezogen auf die tensidhaltige Formulierung betragen.

### Beispiele

### Viskosität

Die Viskositätsmessungen erfolgen mit dem erfindungsgemäß einzusetzenden C12/14 N-(3-Dimethylamino)-Propyl-N-Glucamid (C12/14-DMAP-GA) und den korrespondierenden Säureaddukten.

### Meßgerät und -bedingungen: Brookfield Viskosimeter RTV-DV II, 20 Ulmin, 20°C

Es wurden die Viskositäten von binären Tensidsystemen bestehend aus Lauroylethersulfat (LES) und C12/14 DMAP-GA in Abhängigkeit von:
i) Gesamt-WAS (WAS = Waschaktive Substanz, ergibt sich hier aus Summe LES + DMAP-GA)
ii) Verhältnis LES : DMAP-GA
iii) pH-Wert
iv) NaCl-Gehalt
bestimmt. Ethersulfate, insbesondere LES, sind die bevorzugten Cotenside.

**Tabelle 1:**

| Ergebnisse der Viskositätsmessungen von binären Tensidsystemen LES/DMAP-GA | | | | |
|---|---|---|---|---|
| LES : DMAP-GA | WAS [%] | pH | NaCI [%] | Viskosität [mPas] |
| 9:1 | 10 | 5 | 3,0 | 5050 |
| 8:2 | 10 | 5 | 2,0 | 4620 |
| 7:3 | 10 | 5 | 1,0 | 6150 |
| 9:1 | 10 | 6 | 3,5 | 7500 |
| 8:2 | 10 | 6 | 2,0 | 3970 |
| 8:2 | 10 | 6 | 2,5 | 7650 |
| 7:3 | 10 | 6 | 1,0 | 4130 |
| 7:3 | 10 | 6 | 1,5 | 7950 |

| | | | | |
|---|---|---|---|---|
| 9:1 | 10 | 7 | 3,5 | 7200 |
| 8:2 | 10 | 7 | 3,5 | 5000 |
| 8:2 | 10 | 7 | 4,0 | 7000 |
| 7:3 | 10 | 7 | 2,0 | 5100 |
| 7:3 | 10 | 7 | 2,5 | 7000 |
| 9:1 | 10 | 8 | 3,5 | 5300 |
| 8:2 | 10 | 8 | 2,5 | 4950 |
| 8:2 | 10 | 8 | 3,0 | 7550 |
| 7:3 | 10 | 8 | 2,0 | 5800 |

Des weiteren wurden die Viskositäten von binären Tensidsystemen bestehend aus Alkyldiglycolethersulfat-Natriumsalz (Genapol LRO), C12/C14-DMAP GA* Milchsäure, C12/C14-DMAP-GA*HCl, C16/C18-DMAP-GA* Milchsäure und C16/C18-DMAP-GA*HCl bestimmt. Es wurde eingestellt:
- Gesamt-WAS (WAS = Waschaktive Substanz; Summe aus Genapol LRO und DMAP-GA Säureaddukt; WAS = 15 %)
- Verhältnis Genapol LRO : DMAP-GA Säureaddukt = 7:3
- in Abhängigkeit vom Elektrolytzusatz (NaCI)

**Tabelle 2:**

| Ergebnisse der Viskositätsmessung von binären Tensidsystemen Genapol LRO/DMAP-GA-Säureaddukte | | | | |
|---|---|---|---|---|
| NaCl % | C12/C14-DMAP-GA Milchsäure | C12/C14-DMAP GA HCI | C16/C18-DMAP-GA Milchsäure | C16/C18-DMAP-GA HCI |
| ohne | 26000 | | | Gel |
| NaCl | | | | |
| 0,5 | | 2100 | | Gel |
| 1,0 | 15000 | 23000 | 6300 | Gel |
| 2,0 | 21000 | Gel | Gel | Gel |
| 3,0 | 12500 | Gel | Gel | Gel |
| 4,0 | | Gel | Gel | 20000 |
| 5,0 | | Gel | Gel | |
| 6,0 | | 11800 | 14000 | |
| 7,0 | | 7500 | 4500 | |
| Viskositäten in mPas | | | | |

Die Ausprüfung zeigt, daß die erfindungsgemäß einzusetzenden Verbindungen geeignete Verdicker für flüssige wäßrige Tensidsysteme darstellen, welche durch Variation von Cotensid, Einsatzkonzentration, Elektrolytgehalt und pH-Wert in einem breiten Viskositätsbereich gezielt eingestellt werden können und sich für den Einsatz in folgenden Formulierungen eignen:
Flüssige Universalwaschmittel, flüssige Feinwaschmittel, Handgeschirrspülmittel, Klarspüler, Flüssige Reinigungs- und Desinfektionsmittel, Haarshampoos, Syndetseifen, Haarspülungen, Haarfärbemittel, Haarwellmittel, Schaumbäder, Flotationshilfsmittel, Gesichtsreinigungsmittel, Textil- und Faserhilfsmittel, Lederfettungsmittel, Hilfsmittel für die Feststoffentwässerung.

Die Hautfreundlichkeit der Tenside der Formel 1 und Mischungen dieser Tenside mit anderen Tensiden wird durch die Bestimmung des Zeinwertes und des Red-Blood-Cell (RBC)-Wertes bestätigt.

Für DMAP-GA wurde ein Zein-Wert von 39 mg N/100 ml und ein RBC-Wert von 7 % Denaturierung gemessen. Die Werte entsprechen einer sehr guten Hautverträglichkeit.

### Anwendungstechnische Formulierungsbeispiele

### Beispiel 1: Klares Duschgel mit 15 % Wirkstoffgehalt (WAS): Viskosität: 4140 mPas

| Zusammensetzung: | | |
|---|---|---|
| A | DMAP-GA | 4.00 % |
| B | Wasser | 48.85 % |
| C | ® Genapol LRO flüssig | 40.35 % |
| | PEG 400 | 5.00 % |
| | Parfümöl | 0.30 % |
| | Konservierungsmittel | q.s. |
| | Farbstofflösung | q.s. |
| D | Citronensäure | q.s. |
| E | Natriumchlorid | 1.50 % |

### Herstellung:

I A unter Erwärmung in B auflösen
II Die Komponenten von C nacheinander in I einrühren
III Den pH-Wert mit D regulieren, anschließend die Viskosität mit E einstellen

### Beispiel 2: Klares Duschgel mit 16 % Wirkstoffgehalt (WAS) ohne Natriumchloridzusatz Viskosität: 4750 mPas

| Zusammensetzung: | | |
|---|---|---|
| A | DMAP-GA | 6.00 % |
| B | Wasser | 54.50 % |
| C | ® Genapol LRO flüssig | 30.00 % |
| | ® Genagen CAB 818 | 5.00 % |
| | ® Hostapon KCG | 4.00 % |
| | Parfümöl | 0.50 % |
| | Konservierungsmittel | q.s. |
| | Farbstofflösung | q.s. |
| D | Citronensäure | q.s. |

### Herstellung: analog Beispiel 1

### Beispiel 3: Duschbad mit Perlglanz und 16 % Wirkstoffgehalt (WAS) ohne Zusatz von Natriumchlorid Viskosität: 11 300 mPas

| Zusammensetzung: | | |
|---|---|---|
| A | DMAP-GA | 6.00 % |
| B | Wasser | 54.70 % |
| C | ® Genapol LRO flüssig | 35.00 % |
| | ® Genapol TSM | 4.00 % |
| | Parfümöl | 0.30 |
| | Konservierungsmittel | q.s. |
| | Farbstofflösung | q.s. |
| D | Citronensäure | q.s. |

### Herstellung analog Beispiel 1

### Beispiel 4: Klares Antischuppenschampoo mit 12.5 % Wirkstoffgehalt (WAS) ohne Natriumchlorid Viskosität: 13 800 mPas

| Zusammensetzung: | | |
|---|---|---|
| A | DMAP-GA | 5.00 % |
| | ® Octopirox | 0.50 % |
| B | Wasser | 64.20 % |
| C | ® Genapol LRO flüssig | 30.00 % |
| | Parfümöl | 0.30 % |
| | Konservierungsmittel | q.s. |
| | Farbstofflösung | q.s. |
| D | Citronensäure | |

### Herstellung analog Beispiel 1

### Beispiel 5: Klares Antischuppenschampoo mit 12.5 % Wirkstoffgehalt (WAS): Viskosität: 4010 mPas

| Zusammensetzung: | | |
|---|---|---|
| A | DMAP-GA | 2.50 % |
| | ® Octopirox | 0.50 % |
| B | Wasser | 57.10% |
| C | ® Genapol LRO flüssig | 30.00 % |
| | ® Medialan LD | 6.60 % |
| | Parfümöl | 0.30 % |
| | Konservierungsmittel | q.s. |
| | Farbstofflösung | q.s. |
| D | Citronensäure | q.s. |
| E | Natriumchlorid | 3.00 % |

### Herstellung analog Beispiel 1

### Beispiel 6: Klares Antischuppenschampoo mit 12.5 % Wirkstoffgehalt (WAS): Viskosität: 9000 mPas

| Zusammensetzung: | | |
|---|---|---|
| A | DMAP-GA | 2.50 % |
| | ® Octopirox | 0.50 % |
| B | Wasser | 61.35 % |
| C | ® Genapol LRO flüssig | 30.00 % |
| | ® Genapol SBE | 3.35 % |
| | Parfümöl | 0.30 % |
| | Konservierungsmittel | q.s. |
| | Farbstofflösung | q.s. |
| D | Citronensäure | q.s. |
| E | Natriumchlorid | 2.00 % |

### Herstellung analog Beispiel 1

### Beispiel 7: Klares Haarschampoo mit 15 % Wirkstoffgehalt (WAS): Viskosität: 5100 mPas

| Zusammensetzung: | | |
|---|---|---|
| A | DMAP-GA | 5.00 % |
| B | Wasser | 53.70 % |
| C | ® Genapol LRO flüssig | 35.00 % |
| | ® Genapol SBE | 5.00 % |
| | Parfümöl | 0.30 % |
| | Konservierungsmittel | q.s. |
| | Farbstofflösung | q.s. |
| D | Citronensäure | q.s. |
| E | Natriumchlorid | 1.00 % |

### Herstellung analog Beispiel 1

### Beispiel 8: Klares Geschirrspülmittel mit 32.5 % Wirkstoffgehalt (WAS) ohne Chloridzusatz Viskosität: 880 mPas

| Zusammensetzung | | |
|---|---|---|
| A | DMAP-GA | 5.00 % |
| B | Wasser | 53.70 % |
| C | ® Genapol LRO flüssig | 6.00 % |
| | ® Hostapur SAS 60 | 35.00 % |
| | Parfümöl | 0.30 % |
| | Konservierungsmittel | q.s. |
| | Farbstofflösung | q.s. |
| D | Citronensäure | q.s. |

### Herstellung analog Beispiel 1

### Beispiel 11: (Vergleich) Duschbad mit Perlglanz, 14 % WAS Viskosität: 9100 mPas

| Zusammensetzung: | | |
|---|---|---|
| A | ® GENAPOL LRO flüssig (Hoechst AG) | 35.00 % |
| B | C12/14-DMAP-GA x Milchsäure (Hoechst AG) | 7.60 % |
| | ® GENAPOL PGL (Hoechst AG) | 4.00 % |
| | Parfümöl | 0.30 % |
| | Wasser | 53.10 % |
| | Konservierungsmittel | q.s. |
| | Farbstofflösung | q.s. |
| C | Milchsäure | q.s. |

### Herstellung

- I: Die Komponenten von B nacheinander in A einrühren.
- II: Den pH-Wert mit C regulieren.

### Beispiel 12: (Vergleich) Duschgel klar, 14 % WAS Viskosität: 12400 mPas

| Zusammensetzung: | | |
|---|---|---|
| A | ® GENAPOL LRO flüssig (Hoechst AG) | 30.00 % |
| B | C12/14-DMAP-GA x Salzsäure (Hoechst AG) | 8.4 % |
| | ® GENAGEN CAB 818 (Hoechst AG) | 5.00 % |
| | Parfümöl | 0.30 % |
| | Wasser | 56.30 % |
| | Konservierungsmittel | q.s. |
| | Farbstofflösung | q.s. |
| | Salzsäure | q.s. |

### Herstellung

- I: Die Komponenten von B nacheinander in A einrühren.
- II: Den pH-Wert mit C regulieren.

### Beispiel 13: (Vergleich) Antischuppenshampoo klar, 14 % WAS Viskosität: 12600 mPas

| Zusammensetzung: | | |
|---|---|---|
| A | ® OCTOPIROX (Hoechst AG) | 0.50 % |
| B | ® GENAPOL LRO flüssig (Hoechst AG) | 35.00 % |
| C | C12/14-DMAP-GA x Salzsäure (Hoechst AG) | 8.40 % |
| | Parfümöl | 0.30 % |
| | Wasser | 55.80 % |
| | Konservierungsmittel | q.s. |
| | Farbstofflösung | q.s. |
| D | Salzsäure | q.s. |

### Herstellung

- I: A in B lösen.
- II: Die Komponenten von C nacheinander in I einrühren.
- III: Den pH-Wert mit D regulieren.

### Beispiel 14: (Vergleich) Haarshampoo klar, 14 % WAS Viskosität: 6600 mPas

| Zusammensetzung: | | |
|---|---|---|
| A | ® GENAPOL LRO flüssig (Hoechst AG) | 35.00 % |
| B | C12/14-DMAP-GA x Milchsäure (Hoechst AG) | 7.60 % |
| | Parfümöl | 0.30 % |
| | Wasser | 57.10 % |
| | Konservierungsmittel | q.s. |
| | Farbstofflösung | q.s. |
| C | Milchsäure | q.s. |

### Herstellung

- I: Die Komponenten von B nacheinander in A einrühren.
- II: Den pH-Wert mit C regulieren

### Beispiel 15: Vollwaschmittel

| Zusammensetzung: | |
|---|---|
| Alkylsulfat | 12 % |
| Seife | 1 % |
| Fettalkoholoxethylat | 4 % |
| DMAP-GA | 3 % |
| Natriumcarbonat | 6 % |
| Schichtsilikat SKS-6 | 14 % |
| Zeolith | 14 % |
| Natriumcitrat | 5 % |
| Natriumsulfat | 2 % |
| Natriumpercarbonat | 20 % |
| Bleichaktivator | 4 % |
| Polyacrylat (CP-5) | 6 % |
| Enzyme | 1 % |
| Wasser | ad 100 % |

### Beispiel 16: Feinwaschmittel

| Zusammensetzung | |
|---|---|
| Alkylbenzolsulfonat | 14 % |
| Alkylsulfat | 8 % |
| Seife | 2 % |
| Fettalkoholoxethylat | 4 % |
| DMAP-GA | 2 % |
| Natriumcarbonat | 1 % |
| Schichtsilikat SKS-6 | 5 % |
| Zeolith | 40 % |
| Natriumsulfat | 14 % |
| Enzyme | 1 % |
| Wasser | ad 100 % |

### Verzeichnis der eingesetzten Handelsprodukte:

| | |
|---|---|
| ® Genapol LRO flüssig | C₁₂/C₁₈-Alkyldiglycolethersulfat-Natriumsalz (ca. 27 % WAS) |
| ® Hostapur SAS 60 | Sekundäres Alkansulfonat-Natriumsalz (ca. 60 % WAS) |
| ® Genapol SBE | C₁₂/C₁₈-Alkylpolyglycolethersulfosuccinat-Dinatriumsalz (ca. 40 % WAS) |
| ® Medialan LD | Fettsäuresarcosid-Natriumsalz (ca. 30 % WAS) |
| ® Genapol TSM | Alkylethersulfat und Seidenglanzbilder |
| ® Genapol OA 080 | C12/C14-Fettalkoholethoxylat mit 8 EO |
| ® Genagen CAB 818 | Alkylamidopropylbetain (ca. 30 % WAS) |
| ® Hostapon KCG | N-Cocoylglutaminsäure-mono-Natriumsalz (ca. 25 % WAS) |
| PEG 400 | Polyethylenglycol (Molmasse ca. 400) |
| ® Octopirox | 1-Hydroxy-4-methyl-6-(2,4,4-trimethylpentyl)-2(1H)-pyridon, 2-Aminoethanolsalz (Antischuppenmittel) |

## Patentansprüche

1. Verwendung von Verbindungen der Formel 1 als Verdickungsmittel für flüssige, wäßrige Tensidsysteme 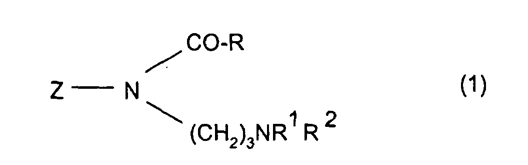 worin R ein aliphatischer Rest mit 8 bis 24 C-Atomen ist, R¹ und R², die gleich oder verschieden sind, Alkyl mit 1 bis 4 C-Atomen oder Hydroxyalkyl mit 2 bis 4 C-Atomen und Z einen linearen Polyhydroxykohlenwasserstoffrest mit mindestens 3 OH-Gruppen, die auch alkoxyliert sein können, bedeuten.

2. Verwendung von Verbindungen nach Anspruch 1, **dadurch gekennzeichnet, daß** R Fettalkyl, R¹ und R² Methyl und Z der Rest eines Zuckeralkohols ist, der sich von einem reduzierenden Mono- oder Disaccharid ableitet.

3. Verwendung von Verbindungen nach Anspruch 2, **dadurch gekennzeichnet, daß** das reduzierende Monosaccharid Glucose ist.

4. Verwendung von Verbindungen nach einem oder mehreren der Ansprüche 1 bis 3, **dadurch gekennzeichnet, daß** sie in einer Menge von 0,5 bis 30 Gewichtsprozent, bezogen auf das gesamte flüssige Tensidsystem, in dem Tensidsystem enthalten sind.

5. Verwendung von Verbindungen nach einem oder mehreren der Ansprüche 1 bis 4, **dadurch gekennzeichnet, daß** zusätzlich ein oder mehrere anionische, kationische, nichtionische und/oder amphotere Tenside verwendet werden.

6. Verwendung von Verbindungen nach einem oder mehreren der Ansprüche 1 bis 5, **dadurch gekennzeichnet, daß** zusätzlich Ethersulfate verwendet werden.

7. Verwendung von Verbindungen nach Anspruch 6, **dadurch gekennzeichnet, daß** das Ethersulfat Lauroylethersulfat ist.

8. Flüssige Feinwaschmittel, Universalwaschmittel, Handgeschirrspülmittel, Klarspüler, flüssige Reinigungs- und Desinfektionsmittel, Syndetseifen, Haarshampoos, Haarspülungen, Haarfärbemittel, Haarwellmittel, Schaumbäder, Gesichtsreinigungsmittel, Textil- und Faserhilfsmittel, Lederfettungsmittel, Flotationshilfsmittel und Hilfsmittel für die Feststoffentwässerung, **dadurch gekennzeichnet, daß** sie Verbindungen nach einem oder mehreren der Ansprüche 1 bis 3 enthalten.

## Claims

1. The use of compounds of the formula 1 as thickeners for liquid, aqueous surfactant systems 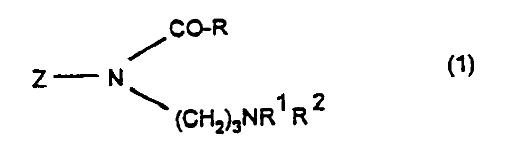 where R is an aliphatic radical having from 8 to 24 carbon atoms, R¹ and R², which are identical or different, are alkyl groups having from 1 to 4 carbon atoms or hydroxyalkyl groups having from 2 to 4 carbon atoms, and Z is a linear polyhydroxyhydrocarbon radical having at least 3 OH groups, which may also be alkoxylated.

2. The use of compounds as claimed in claim 1, wherein R is fatty alkyl, R¹ and R² are methyl, and Z is the residue of a sugar alcohol derived from a reducing mono- or disaccharide.

3. The use of compounds as claimed in claim 2, wherein the reducing monosaccharide is glucose.

4. The use of compounds as claimed in one or more of claims 1 to 3, wherein they are present in the surfactant system in an amount of from 0.5 to 30% by weight, based on the total liquid surfactant system.

5. The use of compounds as claimed in one or more of claims 1 to 4, wherein one or more anionic, cationic, nonionic and/or amphoteric surfactants are additionally used.

6. The use of compounds as claimed in one or more of claims 1 to 5, wherein ether sulfates are additionally used.

7. The use of compounds as claimed in claim 6, wherein the ether sulfate is lauroyl ether sulfate.

8. A liquid light-duty detergent, universal detergent, manual dishwashing detergent, rinse aid, liquid detergent and disinfectant, syndet soap, hair shampoo, hair rinse, hair colorant, hair-waving composition, foam bath, face cleanser, textile and fiber auxiliary, leather fat-liquoring agent, flotation auxiliary and auxiliary for sludge dewatering, which comprise compounds as claimed in one or more of claims 1 to 3.

## Revendications

1. Utilisation de composés de formule 1 comme agents épaississants pour des systèmes d'agents tensioactifs liquides aqueux 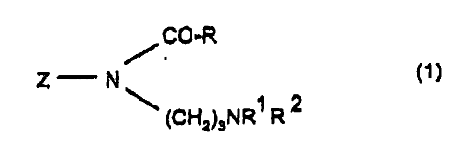 dans laquelle R est un groupe aliphatique ayant de 8 à 24 atomes de carbone, R¹ et R², qui sont identiques ou différents, représentent un groupe alkyle ayant de 1 à 4 atomes de carbone ou hydroxyalkyle ayant de 2 à 4 atomes de carbone et Z représente un groupe polyhydroxyhydro-carboné linéaire ayant au moins 3 groupes OH, qui peuvent aussi être alcoxylés.

2. Utilisation de composés selon la revendication 1, **caractérisée en ce que**, R est un groupe alkyle gras, R¹ et R² sont un groupe méthyle et Z est le reste d'un alcool de sucre, qui dérive d'un mono- ou disaccharide réducteur.

3. Utilisation de composés selon la revendication 2, **caractérisée en ce que** le monosaccharide réducteur est le glucose.

4. Utilisation de composés selon une ou plusieurs des revendications 1 à 3, **caractérisée en ce qu'**ils sont contenus dans le système d'agents tensioactifs en une quantité de 0,5 à 30 % en poids, par rapport au système total d'agents tensioactifs.

5. Utilisation de composés selon une ou plusieurs des revendications 1 à 4, **caractérisée en ce qu'**on utilise en plus un ou plusieurs agents tensioactifs anioniques, cationiques, non ioniques et/ou amphotères.

6. Utilisation de composés selon une ou plusieurs des revendications 1 à 5, **caractérisée en ce qu'**on utilise en supplément des éthersulfates.

7. Utilisation de composés selon la revendication 6, **caractérisée en ce que** l'éthersulfate est l'éthersulfate de lauroyle.

8. Détergents fins liquides, détergents universels, lessive pour le nettoyage de la vaisselle à la main, agents mouillants, agents détergents et de désinfection liquides, savons détergents synthétiques, shampooings capillaires, lotions capillaires, teintures pour cheveux, agents d'ondulation des cheveux, bains moussants, agents de nettoyage facial, adjuvants pour textiles et fibres, agents nourrissants pour le cuir, adjuvants de flottation et adjuvants pour l'assèchement des matières solides, **caractérisés en ce qu'**ils contiennent des composés selon une ou plusieurs des revendications 1 à 3.
